**Europäisches Patentamt**

**European Patent Office**

**Office européen des brevets**

(11) Veröffentlichungsnummer: **0 144 670**
**B1**

(12) **EUROPÄISCHE PATENTSCHRIFT**

(45) Veröffentlichungstag der Patentschrift:
**26.08.87**

(21) Anmeldenummer: **84112721.0**

(22) Anmeldetag: **22.10.84**

(51) Int. Cl.⁴: **A 61 B 6/00,** A 61 B 6/04

(54) **Röntgendiagnostikgerät mit Strahlenfiltern.**

(30) Priorität: **03.11.83 DE 3339775**

(43) Veröffentlichungstag der Anmeldung:
**19.06.85 Patentblatt 85/25**

(45) Bekanntmachung des Hinweises auf die Patenterteilung:
**26.08.87 Patentblatt 87/35**

(84) Benannte Vertragsstaaten:
**DE FR**

(56) Entgegenhaltungen:
**FR - A - 2 104 465**
**FR - E - 93 354**
**US - A - 3 976 889**
**US - A - 4 181 858**

(73) Patentinhaber: **Siemens Aktiengesellschaft Berlin und München, Wittelsbacherplatz 2, D-8000 München 2 (DE)**

(72) Erfinder: **Hüttenrauch, Gerd, Sudetenstrasse 14, D-8524 Uttenreuth (DE)**
Erfinder: **Seubert, Hans-Peter, Kubellohberg 5, D-8551 Heroldsbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Röntgendiagnostikgerät mit Stellmitteln zum Verstellen jeweils eines einer Mehrzahl von Strahlenfiltern in den Strahlengang.

Ein Röntgendiagnostikgerät dieser Art ist in der DE-OS 2 347 178 beschrieben. Bei diesem Röntgendiagnostikgerät erfolgt die Anpassung der Röntgenstrahlung an das jeweilige Aufnahmeobjekt durch Verstellung eines einer Mehrzahl von Strahlenfiltern in die Strahlung vor dem Aufnahmeobjekt. Die Strahlenfilter sind dabei in einer exzentrisch zum Zentralstrahl der Röntgenstrahlung drehbar gelagerten Scheibe angeordnet, die von Hand gedreht werden kann. Ein Parameter, der das jeweils in den Strahlengang zu verstellende Filter bestimmt, ist die Dichte des Untersuchungsobjektes. Die Bedienperson muss bei dem bekannten Röntgenuntersuchungsgerät diese Dichte abschätzen und das entsprechende Filter auswählen.

Der Erfindung liegt de Aufgabe zugrunde, die Filterverstellung zu automatisieren, indem die Dichte des Aufnahmeobjektes automatisch erfasst wird und die Filterverstellung entsprechend erfolgt.

Diese Aufgabe ist demgemäss dadurch gelöst, dass Mittel zur Bildung eines elektrischen Dichtesignals vorhanden sind, das von der Dichte des Untersuchungsobjektes abhängt, und dass die Verstellmittel einen von einer Steuerschaltung angesteuerten Elektromotor enthalten, der das Dichtesignal zugeführt wird und die den Motor dem jeweiligen Dichtesignal entsprechend zur Auswahl des zugehörigen Filters ansteuert. Die Erfindung ist insbesondere für Geräte zur Anfertigung von Mammographieaufnahmen anwendbar, wo eine Kompression der jeweils untersuchten Mamma erfolgt. Das Dichtesignal wird dabei aus der jeweiligen Kompressionskraft und/oder dem jeweiligen Kompressionsweg gebildet.

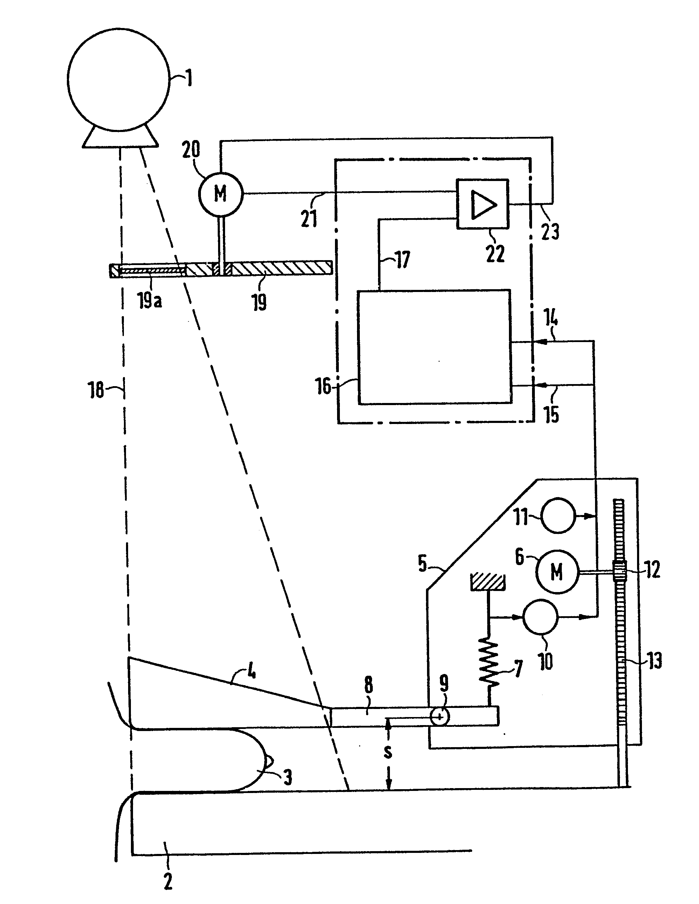

Die Erfindung ist nachfolgend anhand eines in der Zeichnung dargestellten Ausführungsbeispieles näher erläutert.

In der Zeichnung ist ein Röntgendiagnostikgerät für Mammaaufnahmen mit einer Röntgenstrahlenquelle 1 und einem Lagerungstisch 2 für das Aufnahmeobjekt 3 dargestellt. Für die Kompression des Aufnahmeobjektes 3 dient eine Kompressionsvorrichtung mit einem Kompressionstubus 4, der an einem Kompressionswagen 5 gelagert ist und durch einen Elektromotor 6 gegen das Aufnahmeobjekt 3 gedrückt wird. Wenn er an dem Aufnahmeobjekt 3 anstösst und eine bestimmte vorgegebene Kompressionskraft erreicht wird, wird der Motor 6 stillgesezt. Bei am Aufnahmeobjekt 3 anliegendem Kompressionstubus 4 wird eine Feder 7 der jeweiligen Kompressionskraft entsprechend gespannt, die an dem einen Ende eines Hebels 8 angelenkt ist, der um eine Achse 9 schwenkbar gelagert ist und am anderen Ende den Kompressionstubus 4 trägt. Die Feder 7 bildet in Verbindung mit einem Istwertgeber 10 ein der Kompressionskraft entsprechendes elektrisches Signal.

Bei dem dargestellten Gerät wird ferner der jeweilige Kompressionsweg s durch einen Weggeber 11 erfasst und in ein elektrisches Signal umgewandelt, der dem Motor 6 zugeordnet ist. Der Motor 6 verstellt dabei den Kompressionswagen 5 über ein Zahnrad 12, das auf einer gerätefesten Zahnstange 13 abrollt.

Die Istwertsignale für die Kompressionskraft und den Kompressionsweg werden Eingängen 14 und 15 eines Dichterechners 16 zugeführt, der daraus die Dichte des Aufnahmeobjektes 3 und damit das jeweils erforderliche Strahlenfilter bestimmt. Ein dem erforderlichen Filter entsprechendes Signal liegt am Ausgang 17 des Dichterechners 16.

Die vorgesehenen Strahlenfilter können in der in der DE-OS 2 347 178 beschriebenen Weise auf einer exzentrisch zum Zentralstrahl der Röntgenstrahlung 18 drehbar gelagerten Schreibe 19 angeordnet sein. Zur Drehung der Scheibe 19 ist ein Elektromotor 20 vorgesehen, der ein Istwertsignal auf der Leitung 21 bildet, das dem jeweils in den Strahlengang 18 verstellten Filter (bei dem Beispiel 19a) entspricht. Dieses Istwertsignal auf der Leitung 21 wird mit dem Sollwertsignal auf der Leitung 17 verglichen. Stimmt das im Strahlengang befindliche Filter nicht mit dem erforderlichen Filter überein, so liefert ein Verstärker 22 an seinem Ausgang 23 ein Signal, das den Motor 20 so lange einschaltet, bis die Signale auf den Leitungen 17 und 21 übereinstimmen.

Anstelle der Erfassung der Kompressionskraft und des Kompressionsweges kann es bei einfachen Geräten mit fest eingestellter Kompressionskraft ausreichen, nur den jeweiligen Kompressionsweg zu erfassen.

Das Dichtesignal auf der Leitung 17 kann über eine Schnittstelle auch an den die Röntgenstrahlenquelle 1 speisenden Röntgengenerator gemeldet werden und so eine automatische Einstellung der Röntgenröhrenspannung erfolgen.

## Patentansprüche

1. Röntgendiagnostikgerät mit Stellmitteln (19, 20) zum Verstellen jeweils eines einer Mehrzahl von Strahlenfiltern (19a) in den Strahlengang (18), dadurch gekennzeichnet, dass Mittel zur Bildung eines elektrischen Dichtesignales vorhanden sind, das von der Dichtes des Untersuchungsobjektes (3) abhängt, welche von Gebern (7, 10, 11) für die Kompressionskraft und/oder den Kompressionsweg einer Kompressionsvorrichtung (4, 5) für das Untersuchungsobjekt (3) gebildet sind, und dass die Verstellmittel (19, 20) einen von einer Steuerschaltung (16, 22) angesteuerten Elektromotor (20) enthalten, der das Dichtesignal zugeführt wird und die den Motor (20) dem jeweiligen Dichtesignal entsprechend zur Auswahl des zugehörigen Filters (19a) ansteuert.

2. Röntgendiagnostikgerät nach Anspruch 1, dadurch gekennzeichnet, dass das Dichtesignal über den Röntgengenerator die automatische Einstellung der Röntgenröhrenspannung bewirkt.

## Claims

1. An X-ray diagnostic apparatus comprising adjusting means (19, 20) which serve to move one of a plurality of radiation filters (19a) into the beam path (18), characterised in that means are provided which serve to form an electrical density signal dependent upon the density of the object (3) to be investigated, and which are themselves formed by sensors (7, 10, 11) for the compression force and/or the compression path of a compression device (4, 5) for the object (3) to be investigated, and that the adjusting means (19, 20) include an electric motor (20) which is driven by a control circuit (16, 22) which is supplied with the density signal and drives the motor (20) in accordance with the respective density signal in order to select the associated filter (19a).

2. An X-ray diagnostic apparatus as claimed in claim 1, characterised in that the density signal automatically adjusts the X-ray tube voltage via the X-ray generator.

**Revendications**

1. Appareil de radiodiagnostic avec des moyens de réglage (19, 20) pour ajuster respectivement un filtre de rayonnement d'une pluralité de tels filters (19a) dans la trajectoire des rayons (18), caractérisé par le fait que sont prévus des moyens pour former un signal électrique de la densité qui dépend de l'opacité de l'objet à examiner (3), qui sont formés par des générateurs (7, 10, 11) pour la force de compression et/ou de la course de compression d'un dispositif de compression (4, 5) pour l'objet à examiner (3), et que les moyens de réglage (19, 20) comportent un monteur électrique (20) attaqué par un circuit de commande auquel est appliqué le signal de densité, et qui commande le moteur (20) en fonction du signal de la densité pour la sélection du filtre associé (19a).

2. Appareil de radiodiagnostic selon la revendication 1, caractérisé par le fait que le signal de la densité provoque par l'intermédiaire du générateur de rayons, le réglage automatique de la tension du tube radiogène.